(19) Europäisches Patentamt
European Patent Office
Office européen des brevets

(11) **EP 4 674 833 A1**

(12) **EUROPEAN PATENT APPLICATION**
published in accordance with Art. 153(4) EPC

(43) Date of publication:
**07.01.2026 Bulletin 2026/02**

(21) Application number: **23925408.9**

(22) Date of filing: **11.12.2023**

(51) International Patent Classification (IPC):
**C07C 21/18** *(2006.01)*    **C07B 61/00** *(2006.01)*
**C07C 17/25** *(2006.01)*

(52) Cooperative Patent Classification (CPC):
**C07B 61/00; C07C 17/25; C07C 21/18**

(86) International application number:
**PCT/JP2023/044297**

(87) International publication number:
**WO 2024/180856 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2023 JP 2023030188**

(71) Applicant: **AGC INC.**
**Chiyoda-ku,**
**Tokyo 1008405 (JP)**

(72) Inventors:
• **IWASAKI, Hikaru**
**Tokyo 100-8405 (JP)**
• **YAMADA, Taku**
**Tokyo 100-8405 (JP)**
• **OTSUKA, Tetsuo**
**Tokyo 100-8405 (JP)**
• **OKAMOTO, Hidekazu**
**Tokyo 100-8405 (JP)**
• **KAWAGUCHI, Satoshi**
**Tokyo 100-8405 (JP)**

(74) Representative: **Müller-Boré & Partner**
**Patentanwälte PartG mbB**
**Friedenheimer Brücke 21**
**80639 München (DE)**

(54) **COMPOSITION, SYSTEM, CONTAINER CONTAINING COMPOSITION, AND COMPOSITION PRODUCTION METHOD**

(57)    Provided are a composition and application thereof, the composition including trifluoroethylene, and further including 1,1,1-trifluoroethane and 1,1,3,3,3-pentafluoropropene, but not including 1-chloro-2,2-difluoroethylene, (E)-1-chloro-1,2-difluoroethylene, and (Z)-1-chloro-1,2-difluoroethylene, in which a content of the 1,1,1-trifluoroethane is 0.1% by mass or less with respect to a total amount of the composition, and a content of the 1,1,3,3,3-pentafluoropropene is 0.5% by mass or less with respect to a total amount of the composition.

EP 4 674 833 A1

**Description**

Technical Field

The present disclosure relates to a composition, a system, a container containing a composition, and a composition production method.

Background Art

In recent years, trifluoroethylene has garnered attention as a compound with a low global warming potential.

For instance, Patent Literature 1 discloses a composition characterized by including trifluoroethylene and at least one first compound selected from the group consisting of E-1,2-difluoroethylene, Z-1,2-difluoroethylene, 1,1-difluoroethylene, chlorotrifluoroethylene, 1-chloro-2,2-difluoroethylene, E-1-chloro-1,2-difluoroethylene, Z-1-chloro-1,2-difluoroethylene, 1,1,2-trifluoroethane, and methane.

Patent Literature 2 discloses a method of producing trifluoroethylene, characterized by bringing a gas of 1,1,1,2-tetrafluoroethane or a gas of 1,1,1,2-tetrafluoroethane diluted with a dilution gas (provided that a proportion of 1,1,1,2-tetrafluoroethane with respect to a total amount of the dilution gas and 1,1,1,2-tetrafluoroethane is 50% by mole or more) into contact with a first dehydrofluorination reaction catalyst to convert a part of 1,1,1,2-tetrafluoroethane to trifluoroethylene, removing hydrogen fluoride from a reaction product gas obtained by the reaction, and then bringing the reaction product gas from which hydrogen fluoride has been removed in contact with a second dehydrofluorination reaction catalyst to convert at least a part of 1,1,1,2-tetrafluoroethane into trifluoroethylene.

Citation List

Patent Literature

Patent Literature 1: WO2014/178352
Patent Literature 2: WO2015/147063

SUMMARY OF INVENTION

Technical Problem

In a composition containing trifluoroethylene (HFO-1123), there have been cases where it has been required to reduce the content of 1,1,1-trifluoroethane (HFC-143a), which is an impurity, and to suppress the generation of acids.

An object of one embodiment of the present invention is to provide a composition containing HFO-1123, which has a low content of HFC-143a contained as an impurity and suppresses the generation of an acid portion and a method of producing a composition.

An object of another embodiment of the invention is to provide a system including the composition and a composition-containing container that contains the composition.

Solution to Problem

The disclosure encompasses the following aspects.

<1> A composition including trifluoroethylene,

the composition further including 1,1,1-trifluoroethane and 1,1,3,3,3-pentafluoropropene,
but not including 1-chloro-2,2-difluoroethylene, (E)-1-chloro-1,2-difluoroethylene, and (Z)-1-chloro-1,2-difluoroethylene, wherein
a content of the 1,1,1-trifluoroethane is 0.1% by mass or less with respect to a total amount of the composition, and
a content of the 1,1,3,3,3-pentafluoropropene is 0.5% by mass or less with respect to a total amount of the composition.

<2> The composition according to <1>, further including at least one selected from the group consisting of trifluoromethane, vinylidene fluoride, (E)-1,2-difluoroethylene, difluoromethane, (E)-1,3,3,3-tetrafluoropropene, and 1,1,2,2-tetrafluoroethane.

<3> The composition according to <1>, further including trifluoromethane, vinylidene fluoride, (E)-1,2-difluoroethylene, difluoromethane, and 1,1,2,2-tetrafluoroethane.

<4> The composition according to any one of <1> to <3>, further including 1,1,1,2-tetrafluoroethane.

<5> The composition according to <4>, wherein a total content of the trifluoroethylene and the 1,1,1,2-tetrafluoroethane is 80% by mass or more with respect to the total amount of the composition.

<6> The composition according to any one of <1> to <5>, wherein a content of the trifluoroethylene is 3% by mass or more with respect to the total amount of the composition.

<7> A system, including the composition according to any one of <1> to <6> and a contacting member having a surface that comes into contact with the composition, in which the surface at least partially includes a metal as a component.

<8> A composition-containing container, including the composition according to any one of <1> to <6> and a container including a contacting member having a surface that comes into contact with the composition, in which the surface at least partially includes a metal as a component, wherein the composition is sealed in an accommodated state.

<9> A method of producing a composition, including bringing 1,1,1,2-tetrafluoroethane into contact with a catalyst containing $\alpha$-alumina, thereby producing the composition according to any one of <1> to <6>.

<10> The method of producing a composition according to <10>, wherein the catalyst has an average pore size of 5 nm or more.

Advantageous Effects of Invention

According to one of the embodiments of the invention, a composition containing HFO-1123, which has a low content of HFC-143a contained as an impurity and suppresses the generation of an acid portion, and a method of producing a composition are provided.

According to another embodiment of the invention, a system including the composition and a composition-containing container that contains the composition are provided.

DESCRIPTION OF EMBODIMENTS

In the disclosure, a numerical range indicated using "to" means a range that includes the numerical values before and after "to" as the minimum and maximum values, respectively.

In the disclosure, in which numerical ranges are described in stages, the upper limit or lower limit of a specific numerical range may be replaced with the upper or lower limit value of another numerical range described in stages. In addition, in the numerical ranges described in the disclosure, the upper limit or lower limit of a specific numerical range may be replaced with a value shown in Examples.

In the disclosure, a combination of two or more preferred aspects is a more preferred aspect.

In the disclosure, in a case in which there are a plurality of substances corresponding to each component, the amount of each component refers to the total amount of all these substances, unless otherwise specified.

In the disclosure, the statement that "the composition does not contain a specific component" means that the content of the specific component in the composition is less than 1.0 ppm when the content of the specific component in the composition is measured using gas chromatography.

Specifically, it is measured by the following method. Every sample to be analyzed is in a gaseous state.

A gas chromatograph (product name "Gas Chromatograph 6890 Series", manufactured by Agilent Technologies), a column (product name "DB-1", with a length of 60 m, a diameter of 250 $\mu$m, and a filter thickness of 1 $\mu$m), and an FID detector are used. Using a data system (product name "OpenLab", manufactured by Agilent Technologies), the content of a specific component is calculated from the peak area, which is the analysis result. The measurement conditions are as follows.

· Carrier gas: Helium
· Injection temperature: 240°C
· Sample injection volume: 0.5 mL
· Split ratio: 60/1
· Linear velocity: 35.9 cm/sec
· Measurement start: Temperature: -30°C; holding time: 10 minutes
· Heating rate: 10°C/min
· End of measurement: Temperature: 240°C; holding time: 20 minutes
· Detection temperature: 250°C

[Composition]

The composition of the disclosure further includes HFO-1123. The composition further includes HFC-143a and 1,1,3,3,3-pentafluoropropene (HFO-1225zc) but does not include 1-chloro-2,2-difluoroethylene (HCFO-1122), (E)-1-chloro-1,2-difluoroethylene (HCFO-1122a(E)), and (Z)-1-chloro-1,2-difluoroethylene (HCFO-1122a(Z)). The content of HFC-143a is 0.1% by mass or less with respect to the total amount of the composition. The content of HFO-1225zc is 0.5% by mass or less with respect to the total amount of the composition.

In the composition of the disclosure, the content of HFC-143a as an impurity is low, at 0.1% by mass or less with respect to the total amount of the composition. HFC-143a has a high 100-year global warming potential (GWP) of 5,810, as stated in the Sixth Assessment Report (AR6) of the Intergovernmental Panel on Climate Change (IPCC). Therefore, by reducing the content of HFC-143a as an impurity, for example, it is possible to reduce the GWP. In addition, since HFC-143a and HFO-1123 have similar boiling points, it is difficult to separate HFC-143a and HFO-1123 by refining completely, and a low content of HFC-143a as an impurity is advantageous for subsequent use. Since the composition of the disclosure does not contain HCFO-1122, HCFO-1122a(E), or HCFO-1122a(Z), it is possible to suppress the generation of the acid portion that accompanies the elimination of chlorine atoms. Furthermore, by suppressing the generation of the acid portion, corrosion is less likely to occur when the composition of the disclosure is in contact with a metal over an extended period.

In addition, in the method of producing HFO-1123 by the dehydrofluorination reaction of HFC-134a, HFO-1225zc is produced as an impurity. Since HFO-1225zc forms an azeotropic composition with hydrogen fluoride, when the content of HFO-1225zc is high, for example, it becomes difficult to separate hydrogen fluoride produced in the production of HFO-1123 from the composition, and the content of hydrogen fluoride also increases. The increase in the hydrogen fluoride content results in an increase in the acid content in the composition. In the composition of the disclosure, the content of HFO-1225zc is 0.5% by mass or less with respect to the total amount of the composition, such that the content of hydrogen fluoride that may be contained in HFO-1225zc can be reduced. By suppressing the acid content, corrosion is less likely to occur when the composition of the disclosure is in contact with metal for an extended period.

Meanwhile, Patent Literature 1 discloses a method of producing HFO-1123 by hydrogen reduction of chlorotrifluoroethylene (CTFE). The crude product and distillate 2 obtained by this production method contain any one of HCFO-1122, HCFO-1122a(E), or HCFO-1122a(Z). However, the crude product and distillate 2 obtained by this production method do not contain HFO-1225zc.

Patent Literature 1 also discloses a method of producing HFO-1123 by thermal decomposition of a mixture of chlorodifluoromethane (HCFC-22) and chlorofluoromethane (HCFC-31). The crude product obtained by this production method contains any one of HCFO-1122, HCFO-1122a(E), or HCFO-1122a(Z). However, the crude product and distillate 2 obtained by this production method do not contain HFO-1225zc.

In the production method described in Patent Literature 1, HFO-1225zc is not produced, and therefore an increase in the acid content based on HFO-1225zc is not anticipated. Therefore, Patent Literature 1 does not include any description that focuses on the content of HFO-1225zc.

Furthermore, in the production method disclosed in Patent Literature 2, $\gamma$-alumina is used as a catalyst, resulting in high reactivity in the dehydrofluorination reaction and a tendency for the reverse reaction to occur, leading to the by-production of vinylidene fluoride (VdF). An addition reaction of hydrogen fluoride with VdF is likely to occur, resulting in a high content of HFC-143a. Patent Literature 2 does not mention a point that the content of HFC-143a is 0.1% by mass or less.

(HFO-1123)

The composition of the disclosure contains HFO-1123. The composition of the disclosure is preferably produced by a production method in which 1,1,1,2-tetrafluoroethane (HFC-134a) is brought into contact with a catalyst containing $\alpha$-alumina (hereinafter also referred to as the "production method A"). HFO-1123 is obtained as a result of the contact between HFC-134a and $\alpha$-alumina.

The composition of the disclosure may be a crude product (a product before refining, such as distillation) obtained by a method of producing HFO-1123, or a refined product obtained after subjecting the crude product to refining, such as distillation.

The content of HFO-1123 is not particularly limited. However, it is preferably 3% by mass or more, more preferably 4% by mass or more, and still more preferably 5% by mass or more with respect to the total amount of the composition. The upper limit of the content of HFO-1123 is, for example, 99.9% by mass.

In the crude product (outlet gas before refining) in the production of HFO-1123, the content of HFO-1123 is preferably from 3% to 15% by mass, more preferably from 4% to 15% by mass, and still more preferably from 5% to 15% by mass from the viewpoint of carrying out the reaction within an appropriate temperature range so as to maintain the selectivity of HFO-1123.

In the refined product obtained after refining the crude product in the production of HFO-1123, the content of HFO-1123 is preferably 99.0% by mass or more, and more preferably 99.5% by mass or more.

To produce HFO-1123 with a content of 3% by mass or more, a certain degree of conversion rate is required in the production method A. For example, as long as the conversion rate is 1.5% or more, the content of HFO-1123 can be 3% by mass or more.

(HFC-143a)

The composition of the disclosure contains HFC-143a. In the production method A, HFC-143a is obtained as a by-product.

In the disclosure, the content of HFC-143a is 0.1% by mass or less with respect to the total amount of the composition. Since the content of HFC-143a is 0.1% by mass or less, the GWP of the entire composition can be reduced.

The content of HFC-143a is more than 0% by mass, and preferably 0.001% by mass or more.

Furthermore, the content of HFC-143a is preferably 0.1% by mass or less, more preferably, still more preferably 0.09% by mass or less, and even more preferably 0.08% by mass or less.

As described above, HFC-143a is a by-product of the method of producing HFO-1123. However, since HFC-143a and HFO-1123 have similar boiling points, it is challenging to completely separate HFC-143a and HFO-1123 by refining, and a large-scale refining facility is required. Therefore, from the viewpoint of production efficiency, the content of HFC-143a is preferably 0.001% by mass or more

(HFO-1225zc)

The composition of the disclosure contains HFO-1225zc. In the production method A, HFO-1225zc is obtained as a by-product.

In the disclosure, the content of HFO-1225zc is 0.5% by mass or less with respect to the total amount of the composition. Since the content of HFO-1225zc is 0.5% by mass or less, it can prevent the content of the acid portion from increasing.

The content of HFO-1225zc is more than 0% by mass, and preferably 0.0001% by mass or more.

Furthermore, from the perspective of reducing the exposure of HFO-1225zc, the content of HFO-1225zc is preferably 0.5% by mass or less, more preferably 0.25% by mass or less, still more preferably 0.1% by mass or less, and particularly preferably 0.05% by mass or less.

(HFC-134a)

In the production method A, HFC-134a is used as a raw material. Since the forward reaction of producing HFO-1123 from HFC-134a and the reverse reaction of returning from HFO-1123 to HFC-134a also occur, the composition of the disclosure preferably contains HFC-134a.

In a case in which HFC-134a is further contained, the total content of HFO-1123 and HFC-134a is preferably 80% by mass or more, more preferably 90% by mass or more, still more preferably 95% by mass or more, and particularly preferably 99% by mass or more with respect to the total amount of the composition. The upper limit of the above-described total content is, for example, 99.9% by mass.

In the crude product (outlet gas before refining) in the production of HFO-1123, the content of HFC-134a is preferably 65% by mass or more, more preferably 75% by mass or more, still more preferably 80% by mass or more, and particularly preferably 85% by mass or more.

In the refined product obtained after refining the crude product in the production of HFO-1123, the content of HFC-134a is preferably 10.0% by mass or less, more preferably 5.0% by mass or less, still more preferably 1.0% by mass or less, and particularly preferably 0.1% by mass or less.

(HFC-23, VdF, HFO-1132(E), HFC-32, HFO-1234ze(E), HFC-134)

The composition of the disclosure may further include at least one selected from the group consisting of trifluoromethane (HFC-23), vinylidene fluoride (VdF), (E)-1,2-difluoroethylene (HFO-1132(E)), difluoromethane (HFC-32), (E)-1,3,3,3-tetrafluoropropene (HFO-1234ze(E)), and 1,1,2,2-tetrafluoroethane (HFC-134).

Furthermore, the composition of the disclosure may further include HFC-23, VdF, HFO-1132(E), HFC-32, and HFC-134.

In the production method A, HFC-23, VdF, HFO-1132(E), HFC-32, and HFC-134 are obtained as by-products. Without refining treatment, the crude product contains HFC-23, VdF, HFO-1132(E), HFC-32, and HFC-134, along with HFO-1123 and HFC-143a.

The total content of at least one selected from the group consisting of trifluoromethane (HFC-23), vinylidene fluoride (VdF), (E)-1,2-difluoroethylene (HFO-1132(E)), difluoromethane (HFC-32), and 1,1,2,2-tetrafluoroethane (HFC-134) is preferably from 0.0001% to 20% by mass with respect to the total amount of the composition.

The total content of HFC-23, VdF, HFO-1132(E), HFC-32, and HFC-134 is preferably from 0.01% to 20% by mass with respect to the total amount of the composition.

(CTFE)

The composition of the disclosure may contain CTFE.

In the production method A, HFC-134a is used as a raw material. HFC-134a can be obtained, for example, by subjecting CTFE to a hydrogenation reaction, a dehydrochlorination reaction, and a hydrogen fluoride addition reaction. The raw material used in the production method A may contain, in addition to HFC-134a, CTFE that may be contained in the production process of HFC-134a.

In the production method A, when CTFE is contained in the raw material, it is not converted into a different compound and remains unchanged in the reaction product.

When the composition of the disclosure contains CTFE, the content of CTFE is preferably from 0.0001% to 20% by mass with respect to the total amount of the composition.

(HCFO-1122, HCFO-1122a(E), and HCFO-1122a(Z))

The composition of the disclosure does not contain HCFO-1122, HCFO-1122a(E), and HCFO-1122a(Z). HCFO-1122, HCFO-1122a(E), and HCFO-1122a(Z) have a CCl bond. The CCl bond is more easily dissociated than the CF bond. When the CCl bond dissociates, an acid portion may be generated due to the elimination of chlorine atoms. Since the composition of the disclosure does not contain HCFO-1122, HCFO-1122a(E), or HCFO-1122a(Z), it is possible to suppress the generation of the acid portion that accompanies the elimination of chlorine atoms. Furthermore, by suppressing the generation of the acid portion, corrosion is less likely to occur when the composition of the disclosure is in contact with a metal member over an extended period.

From the above-described viewpoint, the composition of the disclosure preferably does not contain HCFO-1122, HCFO-1122a(E), and HCFO-1122a(Z).

Similarly, the composition of the disclosure preferably does not contain an olefin compound with a carbon number of 2 having a chlorine atom and two or fewer fluorine atoms. HCFO-1122, HCFO-1122a(E), and HCFO-1122a(Z) correspond to an olefin compound with a carbon number of 2 having a chlorine atom and two or fewer fluorine atoms.

Examples of the olefin compound with a carbon number of 2 having a chlorine atom and two or fewer fluorine atoms include 1,2-dichloro-1,2-difluoroethylene (CFO-1112(E), CFO-1112(Z)) and 1-chloro-2-fluoroethylene (HCFO-1131(E), HCFO-1131(Z)).

[System]

The system of the disclosure preferably includes the composition of the disclosure and a contacting member having a surface that comes into contact with the composition of the disclosure, in which the surface at least partially includes a metal as a component.

The system may include, for example, piping through which the composition passes, a container for accommodating the composition, and a thermal cycle system.

The surface of the contacting member that comes into contact with the composition of the disclosure may at least partially include a metal as a component. It may be composed of a metal, an alloy containing a metal, or a compound containing a metal.

The metal is preferably at least one selected from the group of metals consisting of iron, copper, aluminum, stainless steel, titanium, nickel, zinc, tin, brass, magnesium, chromium, lead, silver, tungsten, and tantalum.

The alloy is preferably an alloy containing at least one metal selected from the above-described group of metals. Examples of the alloy include a nickel-chrome plate, a solder, and a tin plate.

The metal-containing compound is preferably a compound containing at least one metal selected from the above-described group of metals. Examples of the metal-containing compound include alumite sulfate, zinc phosphate, and iron phosphate.

Since the composition of the disclosure does not contain HCFO-1122, HCFO-1122a(E), or HCFO-1122a(Z), it is possible to suppress the generation of the acid portion that accompanies the elimination of chlorine atoms. Furthermore, by suppressing the generation of the acid portion, the contacting member having a surface that comes into contact with the composition of the disclosure, in which the surface at least partially includes a metal as a component, is less susceptible to corrosion.

[Composition-containing Container]

The composition-containing container of the disclosure is a composition-containing container that includes the composition of the disclosure and a contacting member having a surface that comes into contact with the composition of the disclosure, in which the surface at least partially includes a metal as a component, wherein the composition is sealed in an accommodated state.

The composition-containing container of the disclosure may be, for example, a container in which the entire container includes a metal as a component, a container having a multi-layer structure with the innermost layer including a metal as a component, or a container having a metal coating on the surface that comes into contact with the composition of the disclosure.

The preferred aspects of the metal are as described above.

Since the composition of the disclosure does not contain HCFO-1122, HCFO-1122a(E), or HCFO-1122a(Z), it is possible to suppress the generation of the acid portion that accompanies the elimination of chlorine atoms. Furthermore, by suppressing the generation of the acid portion, a container having a surface that comes into contact with the composition of the disclosure, in which the surface at least partially includes a metal as a component, is less susceptible to corrosion, and thus can store the compositions of the disclosure for an extended period.

[Method of Producing Composition]

In the method of producing a composition of the disclosure, it is preferable to bring HFC-134a into contact with a catalyst containing α-alumina so as to produce the composition of the disclosure.

(Catalyst)

The catalyst contains α-alumina.

The presence of α-alumina in the catalyst can be confirmed by the diffraction pattern obtained using an X-ray diffraction method or X-ray diffractometer (XRD). For XRD, a commercially available device, for example, "SmartLab" manufactured by Rigaku Corporation, can be used. When peaks at d = 26.62, 35.21, 37.85, 43.43, 52.65, and 57.61 Å are present in the diffraction pattern, it can be determined that α-alumina is contained. This analysis should be performed on the catalyst immediately before contact with HFC-134a or on a catalyst that has been subjected to the same conditions as the catalyst immediately before contact with HFC-134a.

The catalyst may contain a compound other than α-alumina. Examples of compounds other than α-alumina include alumina having a crystal structure different from that of α-alumina, and oxides other than alumina. Examples of alumina having a crystal structure different from that of α-alumina include β-alumina, γ-alumina, θ-alumina, η-alumina, boehmite, and gibbsite. Examples of oxides other than alumina include chromium oxide, copper oxide, iron oxide, nickel oxide, magnesium oxide, zinc oxide, and zirconium oxide.

The catalyst may also contain, as a compound other than α-alumina, aluminum oxide fluoride, which is a fluorinated form of α-alumina, or aluminum fluoride.

In particular, from the viewpoint of maintaining the durability of the catalyst, in the diffraction pattern obtained by X-ray diffraction, the catalyst preferably has a diffraction intensity of a peak at d = 43.43 Å among peaks derived from α-alumina that is higher than the diffraction intensity of a peak derived from a compound other than α-alumina, or has only peaks derived from α-alumina. In other words, the catalyst preferably contains α-alumina as the main component.

As described above, the peaks derived from α-alumina correspond to peaks at d = 26.62, 35.21, 37.85, 43.43, 52.65, and 57.61 Å.

The α-alumina may function not only as a catalyst, but also as a carrier while functioning as a catalyst. The α-alumina may be supported on a carrier other than α-alumina.

Examples of carriers include carbon, β-alumina, γ-alumina, zirconia, silica, and titania.

The form of the catalyst is not particularly limited, and may be in the form of a powder, a pellet, or a sphere.

To achieve excellent fillability when filled into a reactor and excellent flowability of a reaction gas, α-alumina is preferably in the form of a molded body, such as a sphere or pellet, from the viewpoint of handling when used for approximately 10 hours.

A molded body is different from a powder and can be obtained, for example, by putting a powder into a mold and compressing it.

Several methods have been proposed for determining the pore structure of a catalyst, and one of the most commonly used methods is measuring the adsorption isotherm through gas adsorption. Nitrogen can be used as a standard adsorption gas for analyzing pores in the micro (less than 2 nm) and mesoporous (from 2 to 200 nm) regions. From the adsorption and desorption isotherms, the parameters specific surface area, average pore size, and pore volume can be obtained.

-Specific Surface Area-

It is generally understood that the higher the surface area accessible to the catalyst, the more frequently the substrate will come into contact with the catalyst surface, and the higher the catalytic activity. It is known that the specific surface area of a catalyst can be increased by decreasing the average pore size and pore volume. For this reason, there are very few cases in which α-alumina, which has the smallest specific surface area among the various alumina crystal structures, is used as a catalyst. However, in contrast to previous understanding, it has been found that increasing the average pore size in α-alumina, which leads to a decrease in the specific surface area, increases the activity of the reaction in the disclosure. The specific surface area of the catalyst can be calculated from the monolayer adsorption of nitrogen based on the Brunauer-Emmett-Teller (BET) theory.

-Average Pore Size-

The average pore size can be calculated based on the Barrett-Joyner-Halenda (BJH) theory.
The average pore size of the catalyst is measured by the following method.
When the average pore size of the catalyst ranges from 2 to 200 nm, it is determined using the BET method and the BJH method, both of which are based on the nitrogen adsorption method. As a measuring device, for example, "3Flex" manufactured by Micrometrics can be used.
The average pore size is calculated using the following Formula, which incorporates the specific surface area (S) determined by the BET method from the adsorption isotherm obtained by the nitrogen adsorption method and the pore volume (V) determined by the BJH method.

$$D = 4V/S$$

D is the average pore size (m) of the sample, V is the pore volume ($m^3/g$) of the sample, and S is the specific surface area ($m^2/g$) of the sample.
Meanwhile, when the average pore size of the catalyst is less than 2 nm, the calculation is performed by the HK method using the Lennard-Jones function.
This measurement should be taken immediately before bringing the catalyst into contact with HFC-134a.
The larger the average pore size, the more pore spaces with larger diameters are present on the surface and inside of the catalyst, improving the diffusivity of HFC-134a and increasing the frequency of contact between the catalyst surface and HFC-134a, which can be expected to enhance reactivity. Moreover, when the average pore size is excessively large, the specific surface area of the catalyst decreases, which tends to suppress the catalytic action.
From the above-described viewpoint, the average pore size of the catalyst is 5 nm or more and preferably from 5 to 200 nm.
The average pore size of the catalyst is preferably 6 nm or more, more preferably 10 nm or more, still more preferably 12 nm or more, and particularly preferably 15 nm or more. From the viewpoint of securing the specific surface area, the average pore size of the catalyst is more preferably 150 nm or less, still more preferably 100 nm or less, particularly preferably 50 nm or less, and most preferably 30 nm or less.

-Pore Volume-

The pore volume can be calculated based on the Barrett-Joyner-Halenda (BJH) theory.
The pore volume affects the diffusivity of the substrate into the pores of the catalyst and the strength of the catalyst. The larger the pore volume, the greater the diffusivity of HFC-134a into the pores, increasing the frequency of contact between the catalyst and HFC-134a, which can be expected to enhance reactivity. Moreover, when the pore volume becomes excessively large, the strength of the catalyst decreases, which tends to reduce the strength of the catalyst.
From the above-described viewpoint, the pore volume of the catalyst is preferably from 0.002 to 1.20 $cm^3/g$.
The pore volume of the catalyst is more preferably from 0.005 $cm^3/g$ or more, still more preferably 0.01 $cm^3/g$ or more, and particularly preferably 0.02 $cm^3/g$ or more. The pore volume of the catalyst is more preferably 1 $cm^3/g$ or less, still more preferably 0.90 $cm^3/g$ or less, and particularly preferably 0.80 $cm^3/g$ or less.

-Bulk Density-

The bulk density of the catalyst is not particularly limited. However, when the bulk density of the catalyst is small, the volume per mass becomes large, and the reactor becomes large, resulting in a decrease in efficiency. On the other hand, when the bulk density of the catalyst is large, the specific surface area and pore volume of the catalyst are reduced.

From the above-described viewpoint, the bulk density of the catalyst is preferably from 0.4 to 1.5 g/mL.

The bulk density of the catalyst is more preferably 0.5 g/mL or more, still more preferably 0.6 g/mL or more, and particularly preferably 0.7 g/mL or more. The bulk density of the catalyst is more preferably 1.4 g/mL or less, still more preferably 1.3 g/mL, and particularly preferably 1.2 g/mL or less.

(Reaction Conditions)

In the method of producing a composition of the disclosure, the raw material gas only needs to contain HFC-134a, and may contain components other than HFC-134a. The raw material gas may consist of only HFC-134a, or may contain an isomer, a disproportionation product, an impurity, and the like obtained during the production of HFC-134a. From the viewpoint of suppressing side reactions and catalyst deactivation, the raw material gas preferably contains, in addition to HFC-134a, an inert gas such as nitrogen, argon, helium, carbon dioxide, or octafluorocyclobutane. The inert gas can dilute a target product and a by-product of hydrogen fluoride. The content of HFC-134a is preferably 60% by mole or more, more preferably 70% by mole or more, still more preferably 75% by mole or more, and particularly preferably 80 by mole or more with respect to the total amount of the raw material gas.

The method of producing a composition of the disclosure may be carried out in the gas phase or in the liquid phase. Since HFC-134a is in a gaseous state at room temperature, it is preferable to bring HFC-134a into contact with the catalyst in the gas phase.

The reactor for bringing HFC-134a into contact with the catalyst is not particularly limited in shape or structure as long as it can withstand the temperatures and pressures described below. The reactor may be, for example, a cylindrical vertical reactor. Examples of materials for the reactor include glass, stainless steel, iron, nickel, and alloys containing iron or nickel as a main component. The reactor may be equipped with a heating means, such as an electric heater, for heating the inside of the reactor.

The catalyst may be accommodated in any of the following forms: fixed bed type, fluidized bed type, or moving bed type. In the case of a fixed bed type, it may be either a horizontal fixed bed type or a vertical fixed bed type.

The reaction may be carried out in a flow manner or in a batch manner.

In a fixed-bed reactor, various molded bodies of catalyst-supporting carriers are filled therein in order to reduce pressure loss of the reaction fluid. In addition, a system in which the catalyst is filled in the same way as in a fixed-bed reactor, moved by gravity, and removed from the bottom of the reactor for regeneration is called a moving bed. In a fluidized-bed reactor, the catalyst bed is operated in such a way that it exhibits fluid-like properties due to the reaction fluid, such that the catalyst particles are suspended in the reaction fluid and move within the reactor. A fixed-bed reactor is preferred because it offers a wide range of options for the shape of the catalyst, and catalyst wear can be minimized. The fixed-bed reactor may be a tubular reactor or a tank reactor, with the tubular reactor being preferred due to the ease of controlling the reaction temperature. Furthermore, a shell-and-tube heat exchanger reaction can be adopted in which a large number of reaction tubes having small diameters are arranged in parallel and a heat transfer medium is circulated on the outside. In a case in which a plurality of reactors are arranged in series, a plurality of catalyst layers are provided. The catalyst layer may have at least one stage, but may have two or more stages.

From the viewpoint of improving the conversion rate, the method of producing a composition of the disclosure is preferably carried out in a flow-type reactor using a fixed-bed reactor (particularly a vertical fixed-bed type reactor).

In the method of producing a composition of the disclosure, HFC-134a is brought into contact with the catalyst at a temperature of preferably from 300°C to 800°C, more preferably from 400°C to 700°C, and still more preferably from 400°C to 600°C. When the contact temperature is 300°C or more, the conversion rate of HFC-134a improves. Meanwhile, when the contact temperature is 800°C or less, the decomposition of HFC-134a can be suppressed. The contact temperature used herein refers to the temperature of the catalyst layer.

Since the dehydrofluorination reaction is generally an endothermic reaction, the decrease in the conversion rate can be suppressed by maintaining the reaction temperature, if appropriate. As the reaction temperature in the catalyst layer increases, the conversion rate of the raw material increases. Therefore, it is preferable to maintain the reaction temperature in the catalyst layer at a desired temperature so as to maintain a high conversion rate. To maintain the reaction temperature in the catalyst layer at a desired temperature, for example, the catalyst layer may be heated from the outside with a heat medium or the like. A catalyst usually deteriorates over time as the reaction proceeds. Even when the conversion rate of the raw material decreases due to catalyst deterioration, the decrease in the conversion rate can be suppressed by heating the catalyst layer with a heat medium or the like and maintaining or increasing the reaction temperature, if appropriate. When the temperature of the catalyst layer is maintained or increased, it is preferable to limit the temperature increase to 50°C or less so as to prevent rapid catalyst deterioration.

At the beginning of the reaction, the reaction zone begins at the introduction point of the raw material gas. When the catalyst at the raw material gas introduction portion deteriorates over time as the reaction proceeds, the reaction zone moves downstream in the gas flow direction. Since the low-temperature product gas produced in the reaction zone flows into the vicinity of the downstream side of the reaction zone, the vicinity of the downstream side is usually the coldest in the catalyst layer. In the disclosure, the temperature of the region of the catalyst layer that is the lowest is referred to as the "minimum temperature of the catalyst layer." The temperature from the vicinity of the downstream side to the further downstream side usually increases from the minimum temperature of the catalyst layer with increasing distance from the reaction zone.

In the method of producing a composition of the disclosure, the raw material gas containing HFC-134a may be supplied to the reactor at room temperature. However, it is preferable for the raw material gas, if appropriate, to be heated (preheated) before being supplied to the reactor. When preheating is performed, the raw material gas is preferably heated to a temperature of from 80°C to 600°C before being supplied to the reactor. When preheating is performed at 80°C or more, the internal temperature of the reactor is less likely to decrease, making it easier to achieve the set conversion rate. Moreover, when preheating is performed at 600°C or less, the internal temperature of the reactor is less likely to rise, an undesirable reaction is suppressed, and the selectivity improves.

Since the dehydrofluorination reaction is a reaction in which the number of molecules increases, increasing the pressure makes the forward reaction disadvantageous.

The pressure when bringing HFC-134a into contact with the catalyst is not particularly limited; however, from the viewpoint of improving the conversion rate, it is preferably maintained from -0.05 to 2 MPa, more preferably from -0.01 to 1 MPa, and still more preferably from normal pressure to 0.5 MPa.

In the disclosure, the term "pressure" refers to gauge pressure.

The contact time between HFC-134a and the catalyst is preferably from 0.5 to 100.0 seconds, more preferably from 1.0 to 50.0 seconds, and still more preferably from 2.0 to 20.0 seconds.

The above-described contact time (sec) is calculated using the following Formula.

Contact time (sec) = [Length of catalyst filled in reactor (cm)]/[Linear velocity (cm/sec)]

The linear velocity means the speed at which the raw material gas containing HFC-134a passes through the catalyst per unit of time.

The contact time (g·sec/mL) between HFC-134a and the catalyst is preferably from 1 to 200 g·sec/mL, more preferably from 5 to 175 g·sec/mL, still more preferably from 7 to 150 g·sec/mL, and particularly preferably from 10 to 125 g·sec/mL. When the contact time (g·sec/mL) is 1 g·sec/mL or more, the conversion rate improves. When the contact time (g·sec/mL) is 200 g·sec/mL or less, equipment costs can be suppressed.

The above-described contact time (g·sec/mL) is calculated using the following Formula.

Contact time (g·sec/mL) = [Amount of catalyst filled (g)]/[Flow rate of HFC-134a (mL/sec)]

From the viewpoint of further suppressing the decrease in conversion, it is preferable to bring HFC-134a into contact with the catalyst in the presence of an inert gas. The inert gas is preferably at least one selected from the group consisting of nitrogen, helium, argon, octafluorocyclobutane, and carbon dioxide. Of these, the inert gas is preferably nitrogen.

The molar ratio of HFC-134a to the inert gas in the gas phase is preferably from 0.1 to 30, and more preferably from 0.5 to 25.

Examples

Hereinafter, the disclosure will be described in more detail with reference to the following Examples. However, the disclosure is not limited to the following Examples, as long as they do not depart from the gist of the disclosure.

[Example 1]

<Preparation of Gas A>

A stainless steel (SUS304) reaction tube having an inner diameter of 3.57 cm and a length of 35 cm was filled with 14 mL of α-alumina (product name "C500" manufactured by Nippon Light Metal Company, Ltd., with an average pore size of 19 nm) as a catalyst. The reaction tube, filled with the catalyst, was placed in a tubular electric furnace, and the catalyst was dehydrated by heating to 450°C with nitrogen passing through the reaction tube.

Thereafter, a 0.1/1 (mol/mol) mixed gas of nitrogen/HFC-134a was passed through the reactor for a contact time of 4.4 seconds, thereby carrying out a HF removal reaction for yielding HFO-1123.

The gas from the outlet of the reaction tube was passed through a KOH alkaline trap, and then the composition (gas A) was recovered in a stainless steel container.

The resulting composition contained HFO-1123, HFC-134a, HFC-143a, and HFO-1225zc in the amounts listed in Table 1. The resulting composition also contained HFC-23, VdF, HFO-1132(E), HFC-32, HFO-1234ze(E), and HFC-134.

The resulting composition was free of HCFO-1122, HCFO-1122a(E), and HCFO-1122a(Z).

Furthermore, the resulting composition did not contain any olefin compound with a carbon number of 2 having a chlorine atom and two or fewer fluorine atoms.

[Example 2]

A composition was obtained in the same method as in Example 1, except that α-alumina (product name "SA52124", manufactured by Saint-Gobain, with an average pore size of 25 nm) was used as the catalyst.

The resulting composition contained HFO-1123, HFC-134a, HFC-143a, and HFO-1225zc in the amounts listed in Table 1. The resulting composition also contained HFC-23, VdF, HFO-1132(E), HFC-32, HFO-1234ze(E), and HFC-134.

The resulting composition was free of HCFO-1122, HCFO-1122a(E), and HCFO-1122a(Z).

Furthermore, the resulting composition did not contain any olefin compound with a carbon number of 2 having a chlorine atom and two or fewer fluorine atoms.

[Examples 3 to 5]

The gas A prepared in Example 1 was mixed with HCFO-1122 (manufactured by SynQuest Laboratories, Inc.), thereby preparing a composition with the content of each component as listed in Table 1.

[Example 6]

A stainless steel (SUS304) reaction tube having an inner diameter of 2.27 cm and a length of 30 cm was filled with 40 mL of γ-alumina (product name "N612N", manufactured by JGC Catalysts and Chemicals Ltd.) as a catalyst.

The reaction tube, filled with the catalyst, was placed in a tubular electric furnace, and the catalyst was dehydrated by heating to 450°C with nitrogen passing through the reaction tube.

Thereafter, a 4/1 (mol/mol) mixed gas of nitrogen/HFC-134a was passed through the reactor for a contact time of 10 seconds, thereby carrying out a HF removal reaction for yielding HFO-1123.

The gas from the outlet of the reaction tube was passed through a KOH alkaline trap, and then the composition was recovered in a stainless steel container.

The resulting composition HFO-1123, HFC-134a, and HFC-143a in the amounts listed in Table 1. The resulting composition also contained HFC-23, HFC-134, VdF, and HFO-1132(E). Furthermore, the resulting composition contained no HFO-1225zc (shown as "0" in Table 1).

The resulting composition was subjected to a stability test. The test method is as follows.

(Stability Test)

The prepared composition was poured into a 75 mL stainless steel (SUS304) cylinder until the pressure reached 0.11 MPaG.

The purity of the sealing gas was evaluated for HFO-1123, HFC-134a, HFC-143a, HFO-1225zc, and HCFO-1122 in terms of mass percentage (% by mass) using gas chromatography.

Thereafter, the cylinder after being sealed was heated in an oven set at 50°C.

After 500 hours, the cylinder was removed and the gas in the container was bubbled into 50 mL of ion-exchanged water. The pH of the resulting aqueous layer was measured using a pH meter (model number "D-51" manufactured by HORIBA, Ltd.) and a pH electrode (model number "9615-10D" manufactured by HORIBA, Ltd.).

Furthermore, the wall surface of the cylinder in which the gas was sealed was visually observed, and the change in appearance before and after the test was evaluated according to the following criteria.

    A: No change before and after the test.
    B: The gloss disappeared after the test.
    C: Corrosion was observed after the test.

Table 1 shows the pH and appearance results. In Example 6, the test was not performed. Thus, "-" is entered. Additionally, in Examples 1, 2, and 6, HCFO-1122 was not added and therefore not detected. Thus, "-" is entered.

[Table 1]

| | Content (% by mass) | | | | | Safety Test | |
|---|---|---|---|---|---|---|---|
| Example 1 | HFO-1123 | HFC-134a | HFC-143a | HFO-1225zc | HCFO-1122 | PH | Appearance |
| Example 2 | 5.85 | 93.4 | 0.0327 | 0.0131 | - | 5.65 | A |
| Example 3 | 7.21 | 92.7 | 0.00637 | 0.0152 | - | 5.7 | A |
| Example 4 | 5.76 | 91.3 | 0.032 | 0.0143 | 1.13 | 5.43 | B |
| Example 5 | 5.61 | 89.1 | 0.0312 | 0.0152 | 5.07 | 5.01 | B |
| Example 6 | 5.51 | 87.2 | 0.0298 | 0.0145 | 10.3 | 4.34 | B |
| Example 7 | 32.1 | 66.8 | 0.121 | 0 | - | - | - |

Examples 1 and 2 are working examples, and Examples 3 to 6 are comparative examples.

As shown in Table 1, it was found that the compositions of Examples 1 and 2 are a composition containing HFO-1123, which further contains HFC-143a and HFO-1225zc but does not contain HCFO-1122, HCFO-1122a(E), and HCFO-1122a(Z), and the content of HFC-143a is 0.1% by mass or less and the content of HFO-1225zc is 0.5% by mass or less with respect to the total amount of the composition, which causes the generation of an acid portion to be suppressed.

The compositions of Examples 3 to 5 contained HCFO-1122, and the generation of an acid portion was confirmed.

The composition of Example 6 has a high content of HFC-143a and tends to have a high GWP.

The disclosure of Japanese Patent Application No. 2023-030188, filed on February 28, 2023, is incorporated herein by reference in its entirety. All publications, patent applications, and standards mentioned herein are incorporated herein by reference to the same extent as if each individual publication, patent application, or standard were specifically and individually indicated to be incorporated by reference.

**Claims**

1. composition comprising trifluoroethylene,

   the composition further comprising 1,1,1-trifluoroethane and 1,1,3,3,3-pentafluoropropene,
   but not comprising 1-chloro-2,2-difluoroethylene, (E)-1-chloro-1,2-difluoroethylene, and (Z)-1-chloro-1,2-difluoroethylene, wherein:

   a content of the 1,1,1-trifluoroethane is 0.1% by mass or less with respect to a total amount of the composition, and
   a content of the 1,1,3,3,3-pentafluoropropene is 0.5% by mass or less with respect to the total amount of the composition.

2. The composition according to claim 1, further comprising at least one selected from the group consisting of trifluoromethane, vinylidene fluoride, (E)-1,2-difluoroethylene, difluoromethane, (E)-1,3,3,3-tetrafluoropropene, and 1,1,2,2-tetrafluoroethane.

3. The composition according to claim 1, further comprising trifluoromethane, vinylidene fluoride, (E)-1,2-difluoroethylene, difluoromethane, and 1,1,2,2-tetrafluoroethane.

4. The composition according to claim 1, further comprising 1,1,1,2-tetrafluoroethane.

5. The composition according to claim 4, wherein a total content of the trifluoroethylene and the 1,1,1,2-tetrafluoroethane is 80% by mass or more with respect to the total amount of the composition.

6. The composition according to claim 1, wherein a content of the trifluoroethylene is 3% by mass or more with respect to the total amount of the composition.

7. system, comprising the composition according to any one of claims 1 to 6 and a contacting member having a surface that comes into contact with the composition, the surface at least partially including a metal as a component.

8. A composition-containing container, comprising the composition according to any one of claims 1 to 6 and a container including a contacting member having a surface that comes into contact with the composition, the surface at least partially including a metal as a component,
wherein the composition is sealed in an accommodated state.

9. A method of producing a composition, comprising bringing 1,1,1,2-tetrafluoroethane into contact with a catalyst containing $\alpha$-alumina, thereby producing the composition according to any one of claims 1 to 6.

10. The method of producing a composition according to claim 9, wherein the catalyst has an average pore size of 5 nm or more.

## INTERNATIONAL SEARCH REPORT

| | International application No. |
|---|---|
| | **PCT/JP2023/044297** |

| A. | CLASSIFICATION OF SUBJECT MATTER |
|---|---|

*C07C 21/18*(2006.01)i; *C07B 61/00*(2006.01)i; *C07C 17/25*(2006.01)i
FI:    C07C21/18; C07C17/25; C07B61/00 300

According to International Patent Classification (IPC) or to both national classification and IPC

| B. | FIELDS SEARCHED |
|---|---|

Minimum documentation searched (classification system followed by classification symbols)

C07C21/18; C07B61/00; C07C17/25

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2019/216175 A1 (DAIKIN INDUSTRIES, LTD.) 14 November 2019 (2019-11-14) claims, paragraphs [0023], [0025], [0049], [0062], [0081], [0104], [0112], examples 7, 15, 18, 19 | 1-10 |
| Y | | 1-10 |
| Y | WO 2016/163522 A1 (ASAHI GLASS COMPANY, LIMITED) 13 October 2016 (2016-10-13) in particular, example 8 | 1-10 |
| Y | JP 2019-167283 A (KAWAI LIME INDUSTRY CO., LTD.) 03 October 2019 (2019-10-03) paragraph [0033] | 1-10 |
| Y | WO 2019/156031 A1 (SEKISUI CHEMICAL CO., LTD.) 15 August 2019 (2019-08-15) paragraph [0063] | 1-10 |

☐ Further documents are listed in the continuation of Box C.          ☑ See patent family annex.

| | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 February 2024** | **27 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
**Information on patent family members**

International application No.

**PCT/JP2023/044297**

| Patent document cited in search report | | | Publication date (day/month/year) | Patent family member(s) | | | Publication date (day/month/year) |
|---|---|---|---|---|---|---|---|
| WO | 2019/216175 | A1 | 14 November 2019 | US 2021/0253502 A1 claims, paragraphs [0042], [0044], [0068], [0081], examples 7, 15, 18, 19 | | | |
| | | | | EP 3792237 A1 | | | |
| | | | | CN 112088150 A | | | |
| WO | 2016/163522 | A1 | 13 October 2016 | US 2018/0037524 A1 example 8 | | | |
| | | | | CN 107531592 A | | | |
| JP | 2019-167283 | A | 03 October 2019 | (Family: none) | | | |
| WO | 2019/156031 | A1 | 15 August 2019 | US 2021/0057732 A1 paragraph [0131] | | | |
| | | | | EP 3754755 A1 | | | |
| | | | | CN 111937189 A | | | |
| | | | | TW 201937784 A | | | |

Form PCT/ISA/210 (patent family annex) (July 2022)

15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014178352 A
- WO 2015147063 A
- JP 2023030188 A